# EUROPÄISCHE PATENTSCHRIFT

(11) **EP 4 311 497 B1**
(45) Veröffentlichungstag und Bekanntmachung des Hinweises auf die Patenterteilung: **26.11.2025**
(21) Anmeldenummer: 22187855.6
(22) Anmeldetag: 29.07.2022
(51) Int. Cl.: A61B 6/03, A61B 6/00

(54) **COMPUTERTOMOGRAPHIE-ANLAGE MIT EINEM KABELFÜHRUNGSSYTEM**
COMPUTER TOMOGRAPHY SYSTEM WITH A CABLE GUIDE SYSTEM
INSTALLATION DE TOMOGRAPHIE ASSISTÉE PAR ORDINATEUR AVEC SYSTÈME DE GUIDAGE DES CÂBLES

(43) Veröffentlichungstag der Anmeldung: 31.01.2024
(73) Patentinhaber: Siemens Healthineers AG, 91301 Forchheim (DE)
(72) Erfinder: Gross, Stefan, 92724 Trabitz (DE)
(74) Vertreter: Siemens Healthineers Patent Attorneys

(56) Entgegenhaltungen:
- DE-A1- 102008 035 196
- DE-A1- 102021 202 983
- JP-A- H09 220 223
- US-A1- 2019 090 830
- US-B1- 6 431 751

## Beschreibung

Vorliegend wird eine bewegliche Computertomographie-Anlage mit einem Kabelführungssystem beschrieben, welches zumindest teilweise deckengeführt bzw. oberhalb der Gantry der Computertomographie-Anlage verläuft.

In modernen medizinischen Untersuchungs- und Behandlungseinrichtungen kommen vermehrt bewegliche Computertomographie (CT)-Anlagen zum Einsatz. Die Bewegbarkeit der Anlagen dient vorwiegend dem Zweck, die typischerweise großen und Raum einnehmenden CT-Anlagen zu verstellen, um in unmittelbarer Umgebung des Patienten Platz für medizinisches Personal und/oder weitere Anlagen oder Geräte, die bei einer Untersuchung, einer Behandlung und/oder einer Intervention zum Einsatz kommen, bereit zu stellen. Dabei steht das Wohl und die Sicherheit des Patienten, aber auch des Bedienpersonals und der Maschinen im Vordergrund.

Daneben bietet eine Bewegbarkeit der CT-Anlagen auch die Möglichkeit, sie in verschiedenen Behandlungsräumen einsetzen zu können und so langfristig Investitions- und Haltungskosten zu reduzieren.

Als Stand der Technik sind hierbei die US 2019/090830 A1, US 6 431 751 B1, DE 10 2021 202 983 A1 und JP H09 220223 A zu nennen.

Bekannt ist es, CT-Anlagen entlang von Schienen zu verstellen, sodass die CT-Anlagen entlang der Schienen vordefinierte Positionen einnehmen können. Alternativ dazu sind auch frei bewegliche CT-Anlagen am Markt verfügbar. Während frei bewegliche CT-Anlagen über eine wiederaufladbare On-Board EnergieVersorgung, bspw. in Form eines Lithium-Ionen-Speichers verfügen, besteht bei schienengebundenen Systemen die Herausforderung, die Versorgung leitungsgebunden zu realisieren.

Bei CT-Anlagen, die in verschiedenen, typischerweise zwei, Behandlungsräumen eingesetzt werden, müssen Kabelführungssysteme beweglich und flexibel ausgebildet sein, und zwar so, dass sie Strecken von bis zu 12 m zu überbrücken können. Die mechanische Beanspruchung darf jedoch die Lebensdauer des Kabelführungssystems nicht beeinträchtigen.

Zudem muss sichergestellt werden, dass das Kabelführungssystem selbst bei einer Verstellbewegung der CT-Anlage keine Kollisionen mit dem Patienten, medizinischem Personal bzw. umgebenden Geräten verursachen kann.

Entsprechend sind im Stand der Technik Lösungen für Kabelführungen vorgesehen, die im Boden, typischerweise in der Nähe des Schienensystems angeordnet sind. Hier sind Kollisionen weitgehend ausgeschlossen. Diese Lösungen erfordern jedoch bauliche Voraussetzungen der Krankenhausumgebung und sind daher nicht uneingeschränkt einsetzbar. Zudem sind sie oft nicht auf die Hygiene-Anforderungen einer medizinischen Umgebung angepasst und teuer.

Alternativ sind Lösungen bekannt, bei denen Versorgungsleitungen mittels einer oder mehrerer Energieführungsketten in einem Deckenkasten angeordnet werden. Das Schienensystem verläuft hier parallel zur Längsachse des Deckenkastens. An der Gantry wird eine mitbewegte Vertikalsäule vorgesehen, durch die die Versorgungsleitungen nach unten zum Fuß der Gantry geführt und dort angeschlossen werden. Die Länge des Deckenkastens definiert dabei den maximalen Verstellweg für die Gantry.

Demgegenüber ist es Aufgabe der vorliegenden Erfindung, eine bewegliche CT-Anlage mit alternativen Mitteln für eine Kabelführung bereit zu stellen, die bei hoher Lebensdauer und geringen Baukosten eine höhere Bewegungsflexibilität ermöglichen. Insbesondere ist es Aufgabe der vorliegenden Erfindung, die Bewegungsfreiheit kabel- und schienengebundener CT-Anlagen so weit zu erhöhen, dass sie in verschiedenen Behandlungsräumen mit entgegengesetzter Betriebsrichtung eingesetzt werden können.

Diese Aufgabe wird gelöst durch eine Computertomographie-Anlage gemäß Anspruch 1.

Gemäß einem ersten Aspekt der Erfindung dient das Kabelführungssystem der Versorgung, insbesondere der energetischen Versorgung einer Computertomographie-Anlage. Entsprechend handelt es sich bei einer Versorgungsleitung um ein ElektroKabel. Alternativ oder zusätzlich kann die Versorgungsleitung der Datenkommunikation von bzw. zu der Computertomographie-Anlage dienen. Daten liegen bspw. in Form von Rohdaten oder bereits rekonstruierten Bilddaten vor. Daten können auch Steuerdaten betreffend den Betrieb der Computertomographie-Anlage umfassen. Insofern kann die Versorgungsleitung auch als Datenkabel ausgebildet sein. Die Gantry der Computertomographie-Anlage ist verstellbar ausgebildet, und zwar in einer zur Gantry senkrecht verlaufenden Bewegungsrichtung. Die Gantry weist eine zentrale Öffnung, die Bore, auf, in welcher ein Patient für eine Bildgebung zumindest teilweise positioniert werden kann. Die Patientenlängsachse entspricht bevorzugt der Bewegungsrichtung der Gantry.

Insofern wird im Folgenden ohne Beschränkung der Allgemeinheit von einem Menschen als Patienten ausgegangen. Grundsätzlich kann der Patient auch ein Tier sein.

Das Kabelführungssystem umfasst gemäß dem ersten Aspekt der vorliegenden Erfindung eine an der Gantry angeordnete Vertikalsäule, die von einem Sockel der Computertomographie-Anlage vertikal nach oben verläuft. Die Vertikalsäule endet bodenseitig auf bzw. mit dem Sockel der Gantry und wird folglich mit der Gantry mitbewegt, wenn diese in der Bewegungsrichtung verschoben wird. Die Vertikalsäule weist eine Höhe auf, die größer als die Höhe der Gantry inklusive Sockel ist. Die Vertikalsäule überragt also die Gantry höhenmäßig. Die Vertikalsäule ist so dimensioniert, dass sie wenigstens eine Versorgungsleitung, bevorzugt eine Mehrzahl von Versorgungsleitungen in sich aufnehmen bzw. in sich führen kann. Die Vertikalsäule besteht in Ausführungen aus einen Aluminium-Blech oder aus einem Kunststoff.

Das Kabelführungssystem umfasst weiter einen ersten Gelenkarm sowie einen zweiten Gelenkarm. Der erste Gelenkarm ist an dem oberen, also deckengerichteten Ende der Vertikalsäule oberhalb der Gantry über einen ersten Gelenkpunkt rotierbar angebunden. Mit anderen Worten kann der erste Gelenkarm seine Relativposition zu der Gantry über eine Rotation bzw. über ein Verschwenken um den ersten Gelenkpunkt ändern. Der erste Gelenkarm ist weiter mit dem ebenfalls oberhalb der Gantry angeordneten zweiten Gelenkarm über einen zweiten Gelenkpunkt rotierbar angebunden. Mit anderen Worten kann der erste Gelenkarm auch seine Relativposition zu dem zweiten Gelenkarm über eine Rotation bzw. über ein Verschwenken um den zweiten Gelenkpunkt ändern.

Sowohl die Vertikalsäule als auch der erste und der zweite Gelenkarm sind ausgebildet, wenigstens eine Versorgungsleitung, bevorzugt eine Vielzahl von Versorgungsleitungen, die dann parallel zueinander bzw. nebeneinander angeordnet sind, zumindest teilweise jeweils entlang ihrer Längsachsen zu führen. Wie eingangs mit Bezug zu der Vertikalsäule schon erläutert, bilden die einzelnen Komponenten des Kabelführungssystems jeweils Hohlräume bzw. zumindest teilweise von außen abgeschirmte Innenbereiche aus, die sich jeweils in Längsrichtung der Komponenten erstrecken und in welchen die wenigstens eine Versorgungsleitung aufgenommen werden kann. Durch den ersten und den zweiten Gelenkpunkt sowie den ersten Gelenkarm ist die Bewegung der Gantry der CT-Anlage vom zweiten Gelenkarm entkoppelt, was eine größere Flexibilität im Hinblick auf die Positionierbarkeit der Gantry bewirkt.

In bevorzugter Ausführung des Kabelführungssystems ist der zweite Gelenkarm weiter über einen deckenständigen dritten Gelenkpunkt oberhalb der Gantry rotierbar angebunden. Der dritte Gelenkpunkt ist folglich nahe bzw. an einer Raumdecke einer Untersuchungs- bzw. Behandlungsumgebung angeordnet. Der dritte Gelenkpunkt kann in Ausführungen ortsfest an der Decke positioniert sein. In alternativen Ausführungen ist auch die Position des dritten Gelenkpunktes verstellbar, vorzugsweise in einer Richtung parallel zur Bewegungsrichtung der Gantry verstellbar ausgebildet, wie weiter unten noch genauer erläutert wird.

Der zweite Gelenkarm ist also mittel- oder unmittelbar mit der Raumdecke über den dritten Gelenkpunkt rotierbar angebunden. Mit anderen Worten kann der zweite Gelenkarm seine Relativposition zu der Raumdecke über eine Rotation bzw. über ein Verschwenken um den dritten Gelenkpunkt ändern.

Durch das Vorsehen eines dritten Gelenkpunktes am zweiten Gelenkarm kann die Positionierungsfreiheit für die CT-Gantry weiter erhöht werden. Über den ersten und den zweiten Gelenkarm, die jeweils gegeneinander bzw. gegen die Gantry bzw. die Raumdecke verschwenkt werden können, ist die Bewegung der Gantry maximal von der Raundecke entkoppelt. Insbesondere ermöglicht diese Entkopplung eine Rotation der Gantry um eine Vertikalachse, die durch das Iso-Zentrum der Gantry verläuft.

Die beiden Gelenkarme werden in Ausführungen der Erfindung auf Stahlblech hergestellt, wobei der Herstellungsprozess insbesondere ein Lasern und ein Biegen umfasst. Die Herstellung muss aber nicht auf diese beiden Schritte beschränkt sein. Damit sind die Gelenkarme vergleichsweise kostengünstig in der Herstellung und weisen ein geringes Gewicht auf, was die Anforderungen an die Auslegung der Antriebseinheiten reduziert.

Die wenigstens eine Versorgungsleitung ist in Ausführung der Erfindung in der Vertikalsäule und an dem ersten, zweiten und/oder dritten Gelenkpunkt in einer Energiekette geführt. Die Energiekette bildet einen zusätzlichen mechanischen Schutz für die wenigstens eine Versorgungsleitung. Insbesondere an den Gelenkpunkten verläuft die wenigstens eine Versorgungsleitung zumindest abschnittsweise außerhalb der Komponenten des Kabelführungssystems. Insbesondere sind an den Gelenkpunkten Abschnitte der wenigstens einen Versorgungsleitung vorgesehen, die zumindest in einigen Relativstellungen der Gelenkarme bzw. zueinander bzw. zu Gantry oder Raumdecke jeweils eine sogenannte Reserve-Schlaufe bilden. Zum Schutz dieser Reserve-Schlaufe der wenigstens einen Versorgungsleitung wird an den genannten Positionen eine Energiekette angebracht. Diese verhindert bspw. eine Beschädigung der Versorgungsleitung durch Aufprall oder Zusammenstoß, verhindert aber auch ein übermäßiges, zu Beschädigungen führendes Verbiegen oder Knicken der Versorgungsleitung.

In Ausführungen des Kabelführungssystems verlaufen bzw. liegen bzw. bewegen sich der erste und der zweite Gelenkarm in einer Ebene. Das heißt, der erste, der zweite und der dritte Gelenkpunkt ermöglichen jeweils eine Rotation bzw. ein Verschwenken um parallel zueinander angeordnete Rotationsachsen. Entsprechend einer bevorzugten Ausführungsvariante liegen die beiden Gelenkarme in einer Horizontalebene, sind also parallel zu einem Untergrund oder der Raumdecke angeordnet. Die Gelenkpunkte können entsprechend besonders einfach ausgebildet sein, da sie jeweils nur einen Bewegungsfreiheitsgrad für die Verschwenkbewegung bereitstellen müssen.

Die Vertikalsäule kann aber auch höhenverstellbar ausgebildet sein. Dann können in Ausführungen der erste, zweite und/oder dritte Gelenkpunkt ausgebildet sein, auch eine Verschwenkbewegung um eine Horizontalachse zu erlauben. In diesem Fall kann die Höhe der Vertikalsäule insbesondere nachträglich, also nach einer Installation der CT-Anlage leicht angepasst werden, um bspw. räumlichen Vorgaben einer spezifischen Behandlungsumgebung gerecht zu werden. In anderen Ausführungen wird die Höhe der Vertikalsäule bereits vor einer Montage bspw. auf eine vorherrschende Raumhöhe eingestellt. Es ist dann ausreichend, dass erster, zweiter und/oder dritter Gelenkpunkt jeweils nur einen Freiheitsgrad aufweisen. Die Höhenverstellbarkeit der Vertikalsäule kann bspw. über eine Teleskop-Ausführung realisiert werden. Dabei umfasst die Vertikalsäule bspw. zwei oder mehr Hohl-Profilsegmente, wovon jeweils zwei zumindest teilweise ineinander geführt gelagert sind. Durch Ausfahren der einzelnen Profilsegmente aus dem jeweils tragenden Segment kann folglich eine Längung der Vertikalsäule erreicht werden. Eine Verkürzung kann entsprechend umgekehrt durch Einfahren der Profilsegmente ineinander erzielt werden.

Die Höhenverstellbarkeit der Vertikalsäule dient vorrangig einer Anpassung des Kabelführungssystems an bestehende räumliche Vorgaben, insbesondere eine Raumdeckenhöhe, also einem Ausgleich zwischen der Höhe des dritten Gelenkpunktes und der Vertikalsäulenhöhe.

Der zweite Gelenkpunkt des Kabelführungssystems ist bevorzugt derart ausgebildet, dass er Stellungen erlaubt, in denen der erste Gelenkarm und der zweite Gelenkarm einen Winkel zwischen 10° und 170° einschließen. Die Winkelangabe bezieht sich hierbei auf die beiden Längsachsen der Gelenkarme. Mit anderen Worten erlaubt der zweite Gelenkpunkt Ausrichtungen von erstem und zweitem Gelenkarm, in denen diese fast bzw. im Wesentlichen parallel entweder hintereinander oder nebeneinander angeordnet sind. Die erste Ausrichtung ermöglicht einen vorteilhaft großen Abstand zwischen Vertikalsäule und dem deckengehängten dritten Gelenkpunkt, was die Bewegungsfreiheit für die Gantry erhöht. Die zweitgenannte Ausrichtung realisiert hingegen einen minimalen Footprint der gesamten CT-Anlage. Mit anderen Worten benötigt die CT-Anlage mit dieser Ausrichtung der beiden Gelenkarme einen kleinstmöglichen Grundflächen- bzw. Raumbedarf. Dies ermöglicht vorteilhaft eine kleinere Dimensionierung von Parkflächen für die CT-Anlage, wenn diese außer Betrieb ist.

In weiterer Ausführung des Kabelführungssystems ist der erste Gelenkpunkt derart ausgebildet, dass er Stellungen erlaubt, in denen der erste Gelenkarm und die Gantry einen Winkel zwischen 10° und 160° einschließen. Die Winkelangabe bezieht sich hierbei auf eine vorab zu definierende Nulllage des ersten Gelenkarms. Mittels der besonders breit vorgegebenen Rotationswinkelbereiche des ersten und des zweiten Gelenkpunktes ermöglicht das Kabelführungssystem insbesondere eine Rotation der Gantry um 180°.

In weiterer Ausführung des Kabelführungssystems ist auch der dritte Drehpunkt ausgebildet, dass er Stellungen erlaubt, in denen der zweite Gelenkarm und eine vorab zu definierende Achse an der Raumdecke einen Winkel zwischen 10° und 160° einschließen. Dadurch wird die Bewegungsfreiheit der CT-Anlage weiter erhöht, da somit auch der zweite Gelenkarm ausgehend vom dritten Gelenkpunkt einen breiten Winkelbereich überspannen kann.

Die Längen des ersten und des zweiten Gelenkarms werden bevorzugt so gewählt, dass die Länge des ersten Gelenkarms 65% bis 75%, insbesondere 68& bis 72%, besonders bevorzugt 70% der Länge des zweiten Gelenkarms entspricht. Empirisch konnten die Erfinder feststellen, dass dieses Längenverhältnis der Gelenkarme eine Bewegungsfreiheit der Gantry besonders gut unterstützt. Insbesondere unterstützt dieses Längenverhältnis der Gelenkarme ein rein rotatorische Bewegung der Gantry um eine Vertikalachse durch ihr Isozentrum um 180°.

In besonders bevorzugter Ausführung ist der erste Gelenkarm maximal 1600 mm und der zweite Gelenkarm maximal 2300 mm lang ist. Mit den angegebenen maximalen Längen der Gelenkarme lässt sich mit dem bislang beschriebenen Kabelführungssystem bereits eine maximale Verfahrstrecke für die Gantry von 5600 mm erreichen. Die Längen der beiden Gelenkarme können in Ausführungen auch entsprechend dem obigen Verhältnis verkürzt werden, um das Kabelführungssystem an bauliche Gegebenheiten der Untersuchungsumgebung oder die Dimensionen der CT-Anlage anzupassen.

Das Kabelführungssystem umfasst ferner in bevorzugter Ausführung eine oberhalb der Gantry verlaufende, deckenständige Horizontalsäule, die sich in ihrer Längsachse parallel zu der Bewegungsrichtung der Gantry erstreckt. Dabei ist an der Horizontalsäule ein in Längsrichtung der Horizontalsäule verstellbarer Laufwagen angeordnet, der den dritten Gelenkpunkt trägt. Der dritte Gelenkpunkt, der in vorherigen Ausführungen fest an der Raumdecke positioniert war, lässt sich in dieser Ausführung parallel zur Bewegungsrichtung der Gantry verstellen. Vorteilhaft lässt sich der Laufwagen dabei über im Wesentlichen die gesamte Länge der ebenfalls deckenständigen Horizontalsäule verstellen. Derart verlängert sich die maximale Verfahrstrecke der Gantry mit den maximalen Längen des ersten und zweiten Gelenkarms auf 12 m und ermöglicht dabei komfortabel einen Einsatz der Computertomographie-Anlage in mehreren Behandlungsräumen.

Alternativ oder zusätzlich zu einer höhenverstellbaren Vertikalsäule, wie oben bereits beschrieben, kann auch der Laufwagen ausgebildet sein, die Höhe des dritten Gelenkpunktes zu verstellen, sodass auch mittels des Laufwagens eine Anpassung des Kabelführungssystems an eine vorgegebene Raumdeckenhöhe realisierbar ist.

Die wenigstens eine Versorgungsleitung wird dann ausgehend von dem dritten Gelenkpunkt am Laufwagen wenigstens teilweise in wenigstens einer, bevorzugt zwei Energieketten in der Horizontalsäule weitergeführt. Die Energieketten sorgen jeweils, wie eingangs schon beschrieben, für einen mechanischen Schutz der wenigstens einen Versorgungsleitung und verhindern insbesondere ein Verknicken der Versorgungsleitung, wenn der Laufwagen zwischen einem ersten Ende der Horizontalsäule und dem zweiten Ende der Horizontalsäule entlang ihrer Längsachse verstellt wird.

In Ausführungen der Erfindung kann der Laufwagen passiv durch einen in der Gantry bzw. im Sockel der Gantry vorgesehenen Antrieb mitbewegt werden. In anderen Ausführungen kann der Laufwagen alternativ oder zusätzlich über eine eigene Antriebseinheit verfügen, um den Laufwagen aktiv entlang der Horizontalsäule zu bewegen. Dies ist insbesondere von Vorteil, um die CT-Anlage in ihre Park-Position bzw. in ihre Parkstellung zu verbringen, in der der erste und der zweite Gelenkarm bzw. einen Winkel von 10° zueinander einnehmen und im Wesentlichen nebeneinander liegend angeordnet sind.

Die Horizontalsäule weist in Ausführungen der Erfindung eine Länge von maximal 7 m entlang der Bewegungsrichtung der Gantry auf. Mit anderen Worten wird der Laufwagen bei einer Bewegung der Gantry über eine Strecke von maximal 7 m mit verstellt.

Ein weiterer Aspekt betrifft eine Computertomographie-Anlage zur Erzeugung tomographischer Röntgenbilder. Diese umfasst eine Gantry, die in einer zu der Gantry senkrecht verlaufenden Bewegungsrichtung verstellbar ist, sowie ein Kabelführungssystem, welches wie oben bereits beschrieben ausgebildet ist. Die Computertomographie-Anlage ist eingerichtet, tomographische Bilddaten eines Patienten, genauer einer Körperregion eines Patienten, mittels Röntgenstrahlung zu erzeugen. Zu diesem Zweck umfasst die Computertomographie-Anlage in ihrer Gantry Bildgebungskomponenten in Form wenigstens einer Röntgenstrahlenquelle und wenigstens eines gegenüberliegend angeordneten Röntgenstrahlendetektors. Die Röntgenstrahlenquelle ist ausgebildet Röntgenstrahlung zu erzeugen und in Richtung Patienten, der im Isozentrum der Gantry angeordnet ist, auszusenden. Die Röntgenstrahlung wird durch die Gewebeverteilung der untersuchten bzw. abgebildeten Körperregion abgeschwächt und trifft, nachdem sie den Patienten durchsetzt hat, auf den Röntgenstrahlendetektor auf.

Die Bildgebungskomponenten der CT-Anlage sind in der Gantry rotierbar angeordnet, sodass Projektionsdaten aus einer Vielzahl von verschiedenen Winkelstellungen erzeugt werden können. Die CT-Anlage umfasst weiter eine Recheneinheit, die ausgebildet ist, aus einer Vielzahl von erfassten Projektionsdaten ein bevorzugt dreidimensionales tomographisches Röntgenbild der Körperregion zu rekonstruieren.

In Ausführungen kann die CT-Anlage ein Schienensystem umfassen, welches in der oben beschriebenen Bewegungsrichtung der Gantry verläuft. Das Schienensystem kann dabei eine, aber auch zwei oder mehr Schienen umfassen. Die Schienen sind in bevorzugter Ausführung geradlinig ausgebildet. In besonderen Ausführungen erstreckt sich das Schienensystem über eine Länge von bis zu 12 **m.** Diese maximale Verfahrstrecke erstreckt sich besonders vorteilhaft und über zwei Untersuchungsräume einer medizinischen Einrichtung. Bei einer maximalen Verfahrstrecke von 12 m können zwei großzügige Untersuchungsräume mit nur einer CT-Anlage genutzt werden, indem die Gantry der CT-Anlage entlang des Schienensystems verstellt wird. Dabei unterstützt das oben beschriebene Kabelführungssystem, welches in Ausführungen ausgebildet ist, über diese maximale Verfahrstrecke die wenigstens eine Versorgungsleitung mitzuführen.

In vorteilhafter Ausführung ist zwischen den beiden Untersuchungsräumen eine Park-Position vorgesehen, die mittels bspw. Schiebetüren von den Untersuchungsräumen getrennt werden kann, um die CT-Anlage außer Betrieb dort zu positionieren. Diese Parkposition kann in besonders vorteilhafter Ausführung besonders klein bzw. schmal ausgebildet sein, da die beiden Gelenkarme des Kabelführungssystems sehr dicht, fast parallel zueinander positionierbar sind.

In weiterer Ausführung kann die Horizontalsäule eine Rollobandabdeckung an der Längsseite umfassen, an welcher der Laufwagen geführt wird. Die Rollobandabdeckung dient vorwiegend einem Verschluss der Horizontalsäule. Die Rollobandabdeckung kann bspw. aus Kunststoff ausgeführt sein. Die Rollobandabdeckung kann alternativ metallisch ausgebildet sein und verringert derart Leckage-Strahlung zwischen den beiden Untersuchungsräumen.

Die CT-Anlage ist ferner ausgebildet, bei einer Verstellbewegung entlang des Schienensystems eine Rotation der Gantry um eine durch das Isozentrum der Gantry verlaufende Vertikalachse um maximal 180° zu vollziehen. Alternativ dazu kann die Gantry auch ortsfest rotiert werden. Dabei unterstützt wiederum das Kabelführungssystem. Mittels des spezifischen Längenverhältnisses zwischen erstem und zweitem Gelenkarm kann die Gantry sich selbst ohne eine translatorische Bewegung entlang des Schienensystems eine Rotation um die Isozentrumsachse um 180° vollziehen.

Dabei kann die CT-Anlage einen rotatorischen Antrieb bzw. eine Drehlagerung umfassen, welcher zwischen Sockel der CT-Anlage und Gantry wirkt. Mit anderen Worten kann der Sockel ausgebildet sein, starr ausgerichtet und mit dem Schienensystem fest verbunden zu sein. Die Gantry hingegen kann durch den rotatorischen Antrieb gegenüber dem Sockel verdreht werden.

In Folgenden werden die Vorteile der Erfindung nochmal zusammengefasst:
Das Kabelführungssystem ermöglicht eine besonders lange Verfahrstrecke für die Gantry einer CT-Anlage und eignet sich daher besonders für die Anwendung in Zwei-Raum-Umgebungen.

Zusätzlich zu der Verfahrstrecke ermöglicht das Kabelführungssystem eine 180°-Drehung der Gantry um die Vertikalachse durch ihr Isozentrum. Die Drehung der Gantry verbessert die Zugänglichkeit der Gantry, derart, dass der Patient bzw. eine Patientenliege samt Patient in jedem der Untersuchungsräume einer Zwei-Raum-Umgebung von den Außenseiten der Untersuchungsräume her in die Gantry geschoben werden kann, was die Patientenpositionierung und damit den Untersuchungsablauf stark vereinfacht.

Das Kabelführungssystem schafft eine einfache Zugänglichkeit zu der wenigstens einen Versorgungsleitung, alle Leitungen bzw. Kabel lassen sich leicht entnehmen. Dies vereinfacht die Wartung des Systems.

Das Kabelführungssystem benötigt weniger Platz an der Raumdecke, sodass freier Platz für andere, insbesondere interventionelle Bildgebungssysteme, bspw. ein C-Bogensystem, mit einer Verstellrichtung quer zur Bewegungsrichtung der Gantry, geschaffen wird.

Zusätzlich ermöglicht das Kabelführungssystem, eine Schiebetür zwischen den zwei Untersuchungsräumen zu schließen, sowohl im als auch außerhalb des Betriebs der CT-Anlage.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine perspektivische Ansicht eines Kabelführungssystems in einer ersten Ausführung der Erfindung,
- FIG 2: eine perspektivische Ansicht einer in einer Untersuchungsumgebung angeordneten Computertomographie-Anlage samt Kabelführungssystem in einer Ausführung der Erfindung in einer ersten Betriebsstellung,
- FIG 3: die Computertomographie-Anlage nach Figur 2 in einer zweiten Betriebsstellung,
- FIG 4: die Computertomographie-Anlage nach Figur 2 in einer dritten Betriebsstellung, und
- FIG 5: die Computertomographie-Anlage nach Figur 2 in einer vierten Betriebsstellung, und
- FIG 6: die Computertomographie-Anlage nach Figur 2 in einer Parkstellung.

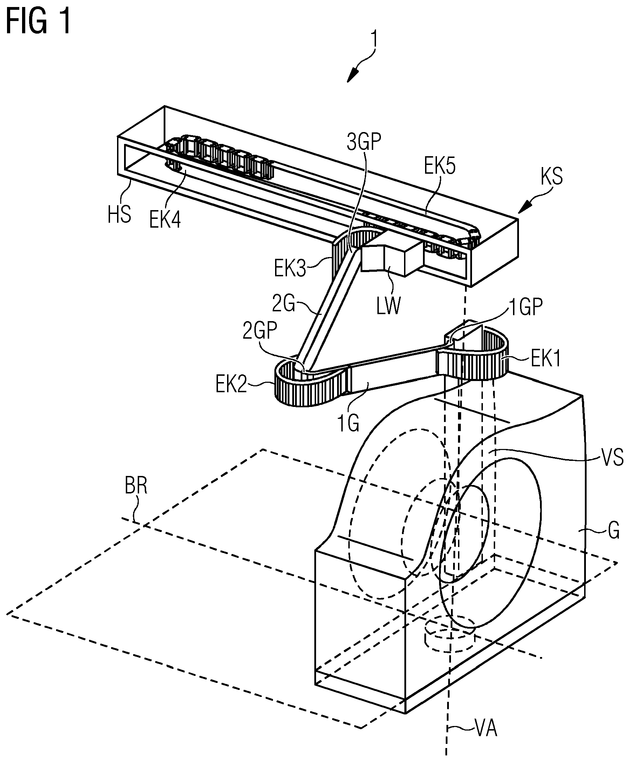

Figur 1 zeigt eine perspektivische Ansicht eines Kabelführungssystems KS in einer ersten Ausführung der Erfindung. Das Kabelführungssystem KS ist für eine Computertomographie-Anlage 1 ausgebildet. Diese weist eine Gantry G auf, die entlang einer zur Gantry G senkrecht verlaufenden Bewegungsrichtung BR verstellt bzw. verschoben werden kann. Das Kabelführungssystem KS umfasst eine hier seitlich bzw. eckständig an der Gantry G angeordnete Vertikalsäule VS, die von einem Sockel der Computertomographie-Anlage 1 vertikal nach oben verläuft. Ferner ist ein erster Gelenkarm 1G sowie ein zweiter Gelenkarm 2G umfasst. Der erste Gelenkarm 1G ist dabei an einem oberen Ende der Vertikalsäule VS oberhalb der Gantry G über einen ersten Gelenkpunkt 1GP rotierbar sowie mit dem ebenfalls oberhalb der Gantry G angeordneten zweiten Gelenkarm 2G über einen zweiten Gelenkpunkt 2GP rotierbar angebunden.

Der erste Gelenkpunkt 1GP bewirkt also, dass der erste Gelenkarm 1G nicht nur oberhalb, sondern auch zumindest teilweise über die Gantry G verschwenkt werden kann. Der zweite Gelenkpunkt 2GP bewirkt, dass eine Relativstellung zwischen erstem und zweitem Gelenkarm 1G, 2G verändert werden kann. Die Vertikalsäule VS weist eine Höhe auf, die sich bis über die Gantry G erstreckt. Die Höhe der Vertikalsäule VS ist dabei so ausgelegt, dass ein Minimal- bzw. Sicherheitsabstand zwischen dem höchsten Punkt der Gantry G und der Unterseite des ersten Gelenkarms 1G eingehalten wird. Die Vertikalsäule VS sowie der erste und zweite Gelenkarm 1G, 2G sind jeweils Hohlräume bzw. innenliegende Bereiche aufweisend ausgebildet, sodass sie wenigstens eine, typischerweise eine Vielzahl von Versorgungsleitungen (nicht gezeigt) in Form von Elektro- oder Datenkabeln zumindest teilweise jeweils entlang ihrer Längsachsen führen können. Mit anderen Worten verlaufen jeweils zumindest Teilabschnitte der wenigstens einen Versorgungsleitung innerhalb der Vertikalsäule VS, dem ersten Gelenkarm 1G und dem zweiten Gelenkarm 1G. Die wenigstens eine Versorgungsleitung erstreckt sich ihrer Länge nach folglich über die Gesamtlänge von Vertikalsäule VS, erstem und zweitem Gelenkarm 1G, 2G und wird durch den Verlauf der genannten Komponenten des Kabelführungssystems KS geführt.

Der zweite Gelenkarm 2G ist in dieser Ausführung weiter über einen deckenständigen dritten Gelenkpunkt 3GP oberhalb der Gantry G rotierbar angebunden. Deckenständig bedeutet in diesem Zusammenhang, dass der dritte Gelenkpunkt 3GP nahe der bzw. an der Decke montiert bzw. befestigt ist. Dadurch kann auch der zweite Gelenkarm 2G nicht nur oberhalb, sondern zumindest auch teilweise über der Gantry G verschwenkt bzw. positioniert werden. Der dritte Gelenkpunkt 3GP ist in dieser Ausführung an einem Laufwagen LW angeordnet. Dieser ist seinerseits an einer oberhalb der Gantry G verlaufenden, deckenständigen, also direkt an der Raumdecke montierten Horizontalsäule HS angebracht. Die Horizontalsäule HS verläuft ihrer Längsachse nach parallel zu der Bewegungsrichtung BR der Gantry G. Der Laufwagen LW ist an einer Längsseite der Horizontalsäule HS ihrer Länge nach verstellbar angeordnet. Derart kann der Laufwagen LW und damit der dritte Gelenkpunkt 3GP über im Wesentlichen die gesamte Länge der Horizontalsäule HS in Bewegungsrichtung BR der Gantry G mitbewegt werden, was die Bewegungsfreiheit für die Gantry G vorteilhaft erhöht. Die Horizontalsäule HS weist vorliegend eine Länge von 7 m auf. Sie kann aber auch, je nach baulicher Voraussetzung der medizinischen Einrichtung, kürzer ausgeführt werden, bspw. 6 m, 5 m oder dergleichen. In dieser Ausführung der Erfindung umfasst der Laufwagen einen Antrieb (nicht gezeigt), der aktiv den Laufwagen LW während einer Verstellbewegung der Gantry G verschiebt. In anderen Ausführungen kann das Verschieben des Laufwagens auch passiv bzw. durch den Antrieb der Gantry G erfolgen.

In weiteren, hier nicht gezeigten Ausführungen ist der dritte Gelenkpunkt 3GP fest an der Raumdecke montiert. Sprich, er lässt sich nicht bewegen. Diese Ausführungen sind besonders gut für Ein-Raum-Anwendungen angepasst.

Der Laufwagen LW ist vorliegend ausgebildet, die Höhe des dritten Gelenkpunktes 3GP zu verstellen. Mit anderen Worten erlaubt der Laufwagen, einen Ausgleich zwischen Raumdeckenhöhe und Höhe der Vertikalsäule VS bzw. den Höhen der drei Gelenkpunkte 1GP, 2GP, 3GP, die immer in einer gemeinsamen Ebene liegen, bevorzugt in einer Horizontalebene, also parallel zur Raumdecke bzw. zum Untergrund. Zu diesem Zweck umfasst der Laufwagen LW in dieser Ausführung einen bspw. teleskopisch ausgebildeten Verstellmechanismus (nicht gezeigt), um durch vertikales Ein- oder Ausfahren von wenigstens einem Teleskop-Segment die Höhe des dritten Gelenkpunktes 3GP einzustellen.

Die wenigstens eine Versorgungsleitung weist vorliegend einen Teilabschnitt auf, der in einer Energiekette oder in mehreren, vorzugsweise zwei Energieketten EK4, EK5 in der Horizontalsäule HS weitergeführt wird. Die Horizontalsäule HS sorgt als weitere Komponente des Kabelführungssystems KS ebenfalls für eine Führung der wenigstens einen Versorgungsleitung.

Die Versorgungsleitung wird nicht nur in der Horizontalsäule HS, sondern auch in der Vertikalsäule VS in einer Energiekette geführt. Zudem sind vorliegend auch an bzw. in den Bereichen des ersten, zweiten und dritten Gelenkpunkts 1GP, 2GP, 3GP jeweils Energieketten vorgesehen, in denen die Versorgungsleitung geführt wird. An diesen Punkten verläuft die wenigstens eine Versorgungsleitung nicht in einem geschützten Hohlraum oder Innenbereich der führenden Komponenten und müssen insbesondere dort mechanisch geschützt werden. Zudem bildet die wenigstens eine Versorgungsleistung an den Gelenkpunkten 1GP, 2GP, 3GP in bestimmten Stellungen der Gelenkarme 1G, 2G und der Gantry G und des Laufwagens LW Reserveschlaufen, die besonders abgesichert werden müssen. Energieketten sorgen allgemein für eine Stabilisierung der wenigstens einen Versorgungsleitung, verhindern Beschädigung bzw. übermäßiges Verknicken oder Verdrehen.

Zusätzlich zu dem Laufwagen LW, der den dritten Gelenkpunkt 3GP in seiner Höhe anpassen kann, ist vorliegend auch die Vertikalsäule VS höhenverstellbar ausgebildet, sodass auch der erste und zweite Gelenkpunkt 1GP, 2GP in ihrer Höhe angepasst werden können, um eine geeignete Betriebsebene für die Gelenkpunkte einzustellen. In diesem Sinne ist in der gezeigten Ausführung zumindest der erste und der dritte Gelenkpunkt 1GP, 3GP ausgebildet, eine Verschwenkbewegung nicht nur um eine Vertikalachse, sondern auch um eine um eine Horizontalachse zu erlauben. Auch die Vertikalsäule VS kann über einen Teleskop-Mechanismus verfügen, der bevorzugt am oberen Ende der Vertikalsäule VS angeordnet ist, welcher durch Ein- und Ausfahren wenigstens eines Teleskop-Abschnitts die Höhe des ersten Gelenkpunktes 1GP einstellt.

Der zweite Gelenkpunkt 2GP ist vorliegend derart ausgebildet, dass er Stellungen erlaubt, in denen der erste Gelenkarm und der zweite Gelenkarm 1G, 2G einen Winkel zwischen 10° und 170° einschließen. Erster und dritter Gelenkpunkt 1GP, 3GP können dadurch fast maximal voneinander (entsprechend der Summe ihrer Einzellängen) entfernt oder minimal voneinander (entsprechend einer Differenz ihrer Einzellängen) entfernt werden, was die Bewegungsfreiheit für die Gantry G weiter erhöht.

Der erste Gelenkpunkt 1GP ist weiter ausgebildet ist, dass er Stellungen erlaubt, in denen der erste Gelenkarm 1G und die Gantry G einen Winkel zwischen 10° und 160° einschließen. Derselbe Winkelbereich gilt ebenfalls für den dritten Gelenkpunkt 3GP. Das Gelenksystem umfassen die beiden Gelenkarme 1G, 2G und die drei Gelenkpunkte 1GP, 2GP, 3GP erlaubt beliebige Stellungen zwischen Gantry G und drittem Gelenkpunkt 3GP. Durch das Vorsehen des Laufwagens LW bzw. der Horizontalsäule HS wird der Bewegungsradius für die Gantry G weiter vorteilhaft erweitert.

Die Länge des ersten Gelenkarms 1G entspricht bevorzugt 65% bis 75%, hier ca. 70% der Länge des zweiten Gelenkarms 2G. Durch Einhaltung dieses Längenverhältnisses kann die Gantry G ortsfest um eine Vertikalachse VA um 180° gedreht werden. Vorliegend erfordern die Dimensionen der Gantry G sowie der Einsatz einer Computertomographie-Anlage 1 in einer Zwei-Raum-Umgebung, dass der erste Gelenkarm 1G 1600 mm und der zweite Gelenkarm 2G 2300 mm lang ist.

Ohne Horizontalsäule HS bzw. Laufwagen LW ermöglicht das Kabelführungssystem KS mit diesen Gelenkarmlängen eine maximale Verfahrstrecke entlang der Bewegungsrichtung BR für die Gantry G von 5600 mm, mit 7 m langer Horizontalsäule HS erhöht sich die maximale Verfahrstrecke auf 12 m.

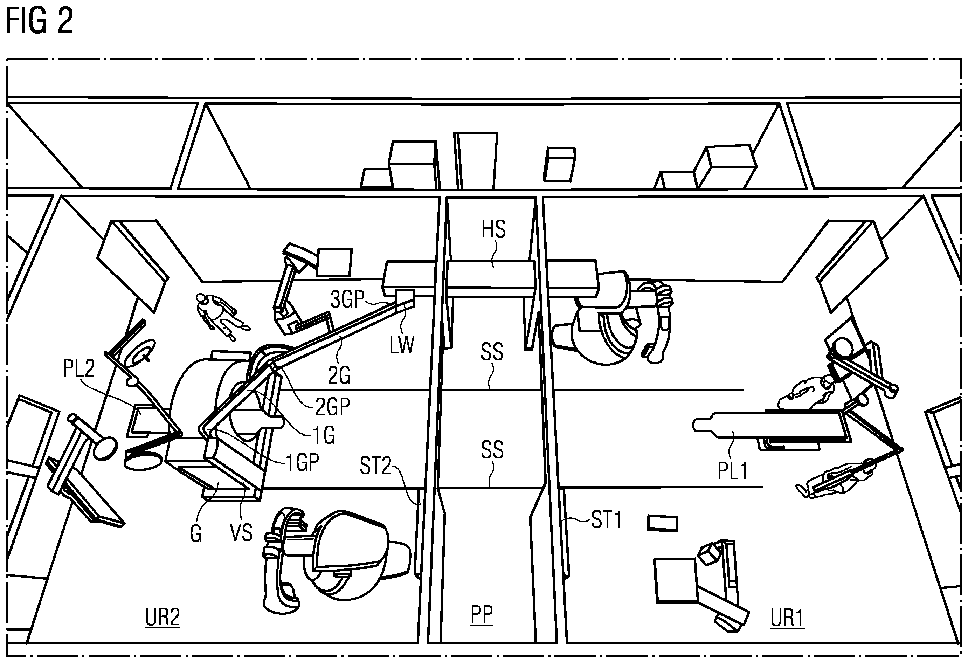

Figur 2 zeigt eine perspektivische Ansicht einer in einer Untersuchungsumgebung angeordneten Computertomographie(CT)-Anlage 1 samt Kabelführungssystem KS in einer Ausführung der Erfindung in einer ersten Betriebsstellung. Die Untersuchungsumgebung ist eine Zwei-Raum-Umgebung umfassend zwei Untersuchungsräume UR1 und UR2. Diese werden von einer Parkposition PP räumlich voneinander getrennt. Wird die CT-Anlage 1 nicht gebraucht bzw. ist sie außer Betrieb, kann sie in die Parkposition PP verbracht und die Untersuchungsräume UR1, UR2 weiter für andere medizinische Anwendungen genutzt werden. Zu diesem Zweck sind in den Untersuchungsräumen UR1, UR2 jeweils auch weitere medizinische Geräte und Anlagen, unter anderem C-Bögen oder Monitore zur in-situ Bildanzeige oder Geräte zur Überwachung physiologischer Funktionen eines Patienten vorgesehen. Des Weiteren sind in jedem Untersuchungsraum UR1, UR2 jeweils Patientenliegen PL1, PL2 angeordnet, die jeweils fest mit dem Boden verbunden, also ortsfest angeordnet sind. Die Liegenbretter sind gegenüber einem Liegensockel verstellbar, insbesondere translatorisch verstellbar, um eine Feinpositionierung des Patienten für eine Bildgebung und/oder einen interventionellen Eingriff zu ermöglichen.

Die Computertomographie-Anlage 1 dient zur Erzeugung tomographischer Röntgenbilder, sowohl in dem ersten als auch in dem zweiten Untersuchungsraum UR1, UR2. Entsprechend umfasst sie eine Gantry G, die in einer zur Gantry G senkrecht verlaufenden Bewegungsrichtung verstellbar ist. Die Bewegungsrichtung BR erstreckt sich hier entlang des Schienensystems SS umfassend zwei Führungsschienen. Die Schienen weisen, wie mit Bezug zu Figur 1 bereits beschrieben, eine Länge von 12 m auf. Ihre Länge und ihr Verlauf definieren den Bewegungsradius der Gantry G.

Die Gantry G ist ferner um eine durch das Isozentrum der Computertomographie-Anlage 1 verlaufende Vertikalachse VA rotierbar ausgebildet. Dies wird durch das oben bereits beschriebene Kabelführungssystem KS umfassend mehrere Komponenten ermöglicht, welches ebenfalls von der CT-Anlage 1 umfasst ist. Insbesondere umfasst das Kabelführungssystem KS eine Horizontalsäule HS mit 7 m Länge, die derart angeordnet ist, dass sie die Parkposition PP überragt und in beide Untersuchungsräume UR1, UR2 hineinragt. Dabei kann eine Rotation ortsfest, also ohne parallele Translation der Gantry G entlang des Schienensystems SS bzw. des Laufwagens LW in paralleler Richtung allein durch verstellen der Gelenkarme 1G, 2G erfolgen. Alternativ kann sie zusammen mit bzw. kontinuierlich parallel zu einer translatorischen Bewegung der Gantry G entlang des Schienensystems SS erfolgen.

In Figur 2 steht die Gantry G im Untersuchungsraum UR2 in der linken Maximalstellung entsprechend des linken Endes des Schienensystems SS. Die Gantry G ist derart rotiert, dass sie mit ihrer Frontseite auf die Außenseite (links) des Untersuchungsraums 2 gerichtet ist. In dieser Stellung ragt die Patientenliege PL2 mit ihrem Liegenbrett in die Bore der Gantry G hinein. In dieser Stellung der Gantry G können tomographische Röntgenbilder eines Patienten erzeugt werden. Um diese Stellung zu erreichen, nimmt das Kabelführungssystem KS eine maximal gestreckte Stellung ein. Gelenkpunkt 2GP ist auf ca. 170° aufgeweitet, sodass erster und zweiter Gelenkarm 1G, 2G im Wesentlichen hintereinander liegen und sich ihre Längen aufaddieren. Beide Gelenkarme 1G, 2G verlaufen nicht über der Gantry G. Der Laufwagen LW befindet sich ebenfalls in seiner linken Maximalstellung in Bezug auf die Horizontalsäule HS. Dennoch sind der dritte und der erste Gelenkpunkt 1GP, 3GP maximal beabstandet. Derart kann über das Kabelführungssystem KS die erforderliche Länge der Versorgungsleitung bis zu einem Anschlusspunkt am unteren Ende der Vertikalsäule VS bereitgestellt werden.

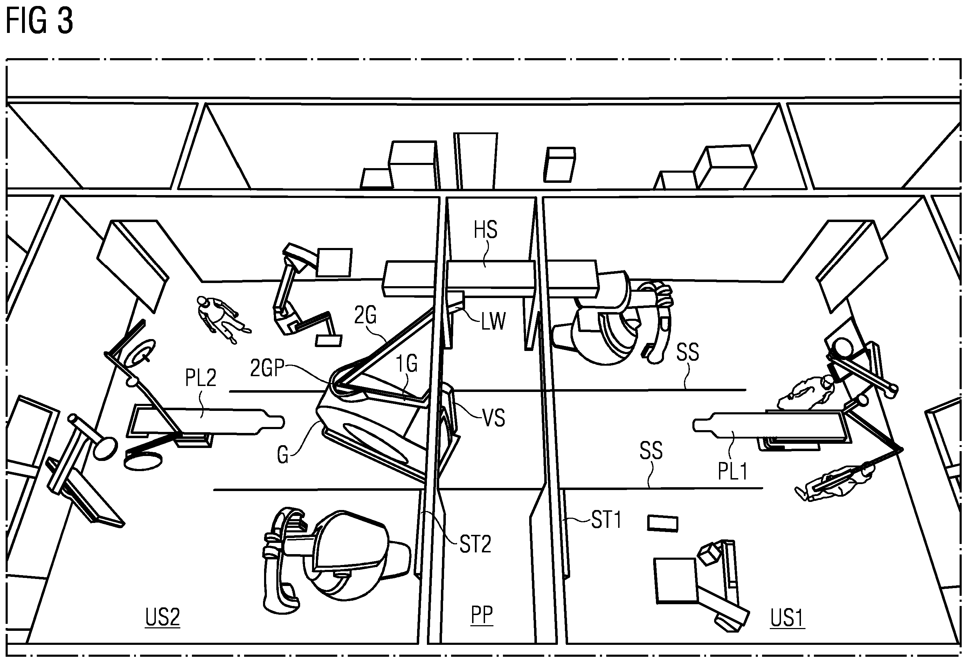

Die Figuren 3 bis 5 zeigen den Übergang der Gantry G von der linken Maximalstellung in die rechte Maximalstellung im gegenüberliegenden Untersuchungsraum UR1.

Figur 3 zeigt die Computertomographie-Anlage 1 entsprechend in einer zweiten Betriebsstellung innerhalb des Untersuchungsraums UR2. Die Gantry G ist bereits hier um ca. 50° entgegen dem Uhrzeigersinn um die Vertikalachse VA gedreht. Der zweite Gelenkpunkt 2GP schließt einen Winkel von ca. 45° zwischen den beiden Gelenkarmen 1G, 2G ein. Der erste Gelenkarm 1G liegt hier vollständig über der Gantry G, so auch der zweite Gelenkpunkt 2GP. Der Laufwagen LW hat eine Position innerhalb der Parkposition PP eingenommen, hat also ca. ein Viertel seines maximalen Verstellwegs entlang der Horizontalsäule HS zurückgelegt.

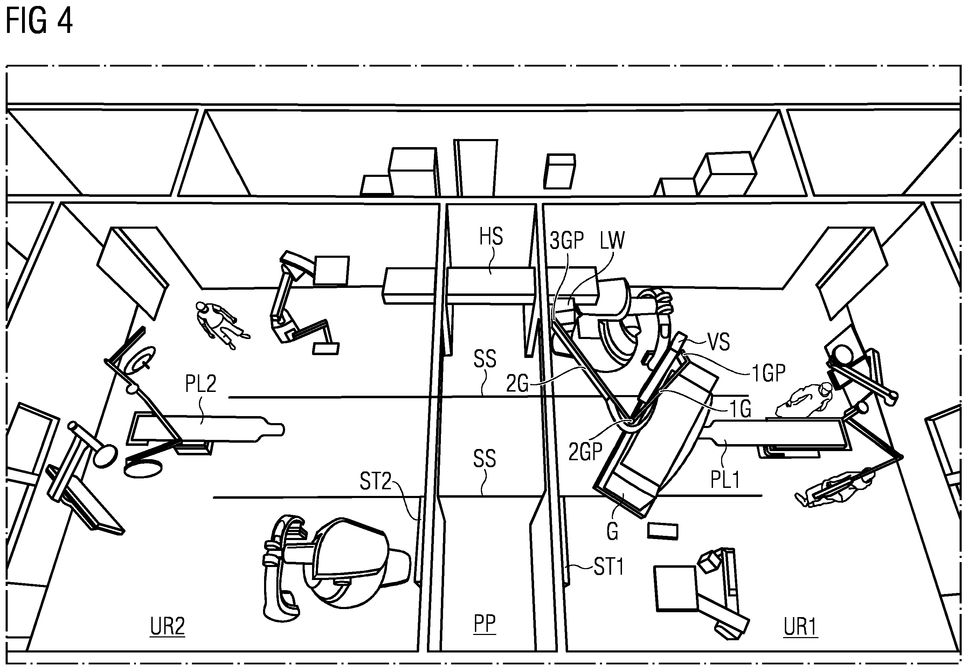

Figur 4 zeigt die Computertomographie-Anlage 1 entsprechend in einer dritten Betriebsstellung innerhalb des Untersuchungsraums UR1. Die Gantry G ist um ca. 150° entgegen dem Uhrzeigersinn um die Vertikalachse VS gedreht. Die Gantry G hat sich weit in den Untersuchungsraum UR1 hineinbewegt. Der zweite Gelenkpunkt 2GP schließt einen Winkel von ca. 80° zwischen den beiden Gelenkarmen 1G, 2G ein. Der erste Gelenkarm 1G liegt auch hier weitgehend über der Gantry G, der zweite Gelenkpunkt 2GP sowie der zweite Gelenkarm 2G nicht. Der Laufwagen LW hat die Parkposition PP verlassen und sich ebenfalls entlang der Horizontalsäule HS nach rechts in den Untersuchungsraum UR1 hineinbewegt. Er hat ca. vier Fünftel seines maximalen Verstellwegs entlang der Horizontalsäule HS zurückgelegt.

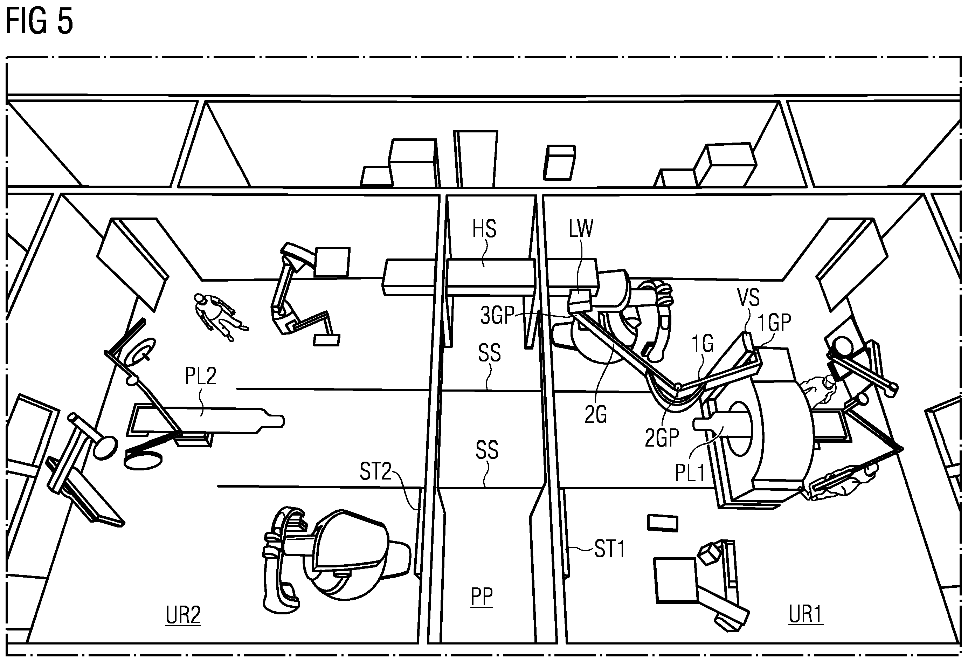

Figur 5 zeigt die Computertomographie-Anlage 1 in einer vierten Betriebsstellung, in der die Gantry G die rechte Maximalstellung im Untersuchungsraum UR1 entsprechend des rechten Endes des Schienensystems SS eingenommen hat. Die Gantry G ist gegenüber der Figur 2 um 180°also vollständig rotiert und sie ist mit ihrer Frontseite auf die Außenseite (rechts) des Untersuchungsraums UR1 gerichtet. In dieser Stellung ragt die Patientenliege PL1 mit ihrem Liegenbrett in die Bore der Gantry G hinein. In dieser Stellung der Gantry G können wieder tomographische Röntgenbilder eines Patienten erzeugt werden. Insbesondere bewirkt die Drehung, dass die Patientenliege in jedem Untersuchungsraum von der Raumaußenseite her in die Bode eingeführt werden und damit umständliche Verstellbewegungen mit der Patientenliege zum Patientenwohl und zur Vereinfachung des Untersuchungsablaufs vermieden werden können. Zudem können die Patientenliegen PL1, PL2 fest in den Untersuchungsräumen UR1, UR2 installiert werden. Um die rechte Maximalstellung zu erreichen, nimmt das Kabelführungssystem KS eine gestreckte, wenn auch nicht maximal gestreckte Stellung ein. Gelenkpunkt 2GP ist auf ca. 105° aufgeweitet, sodass sich die Längen des ersten und zweiten Gelenkarms 1G, 2G weitgehend aufaddieren. Beide Gelenkarme 1G, 2G verlaufen wieder nicht über der Gantry G. Der Laufwagen LW befindet sich in seiner rechten Maximalstellung in Bezug auf die Horizontalsäule HS. Der dritte und der erste Gelenkpunkt 1GP, 3GP sind nicht maximal, aber dennoch sehr weit beabstandet. Auch in dieser Stellung der Gantry G kann das Kabelführungssystem KS die erforderliche Länge der Versorgungsleitung bis zu dem Anschlusspunkt am unteren Ende der Vertikalsäule VS bereitstellen.

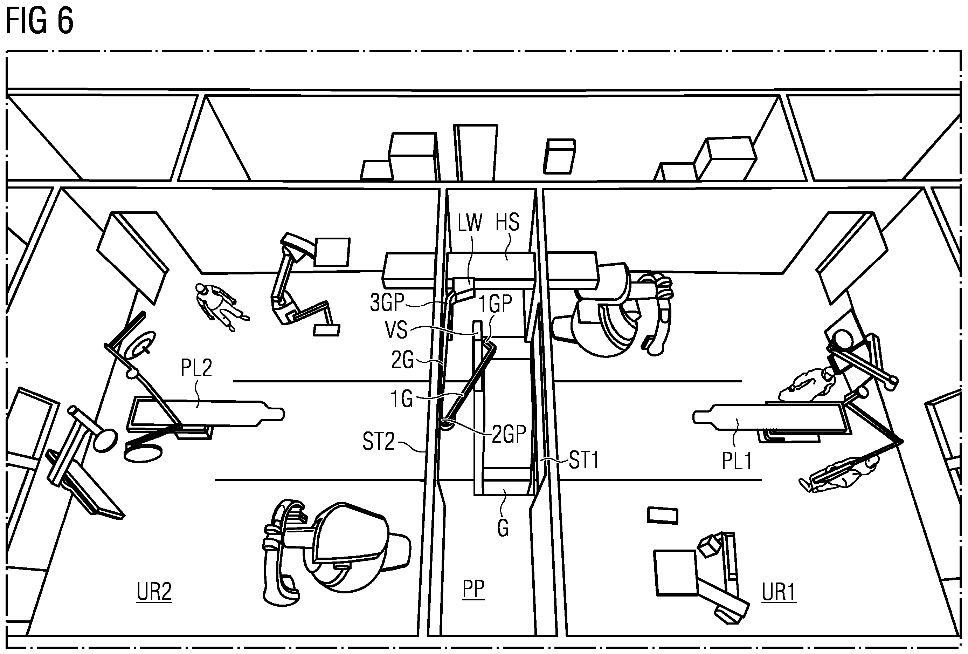

Figur 6 zeigt die Computertomographie-Anlage 1 nach Figur 2 in einer Parkstellung, in welcher die CT-Anlage 1 außer Betrieb ist. Die CT-Anlage 1 nimmt hier die Parkstellung innerhalb der Parkposition PP ein. Bei der Verstellbewegung der Gantry G geöffnete Schiebetüren ST1, ST2 der Parkposition PP können dabei geschlossen werden, sodass jeder Untersuchungsraum UR1, UR2 unabhängig voneinander anderweitig genutzt werden kann.

Die Gantry G nimmt in der Parkstellung eine vorab definierte, maximal rechts in der Parkposition PP liegende Position ein, wobei sie entsprechend der rechten Maximalposition derart rotiert ist, dass ihre Front auf die Außenseite (rechts) von Untersuchungsraum UR1 ausgerichtet ist. Der zweite Gelenkpunkt GP2 schließt hier einen Winkel von 10° zwischen den beiden Gelenkarmen 1G, 2G ein, sodass diese platzsparend im Wesentlichen nebeneinander angeordnet sind. Insbesondere der zweite Gelenkarm 2G verläuft in dieser Stellung im Wesentlichen parallel zur Gantry-Rückseite. Zum Erreichen dieser Parkstellung wird der Laufwagen LW mittels seines Antriebs aktiv an die entsprechende Position entlang seiner Verstellwegs an der Horizontalsäule HS bewegt, um den Winkel von 10° im zweiten Gelenkpunkt 2GP zu erreichen.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Computertomographie-Anlage (1) mit einem Kabelführungssystem (KS), wobei eine Gantry (G) der Computertomographie-Anlage (1) in einer zur Gantry (G) senkrecht verlaufenden Bewegungsrichtung (BR) verstellbar ausgebildet ist,
das Kabelführungssystem (KS) umfassend
- eine an der Gantry (G) angeordnete Vertikalsäule (VS), die von einem Sockel der Computertomographie-Anlage (1) vertikal nach oben verläuft, und
- einen ersten Gelenkarm (1G) sowie einen zweiten Gelenkarm (2G), wobei der erste Gelenkarm (1G) an einem oberen Ende der Vertikalsäule (VS) oberhalb der Gantry (G) über einen ersten Gelenkpunkt (1GP) rotierbar sowie mit dem ebenfalls oberhalb der Gantry (G) angeordneten zweiten Gelenkarm (2G) über einen zweiten Gelenkpunkt (2GP) rotierbar angebunden ist,
wobei die Vertikalsäule (VS) und der erste und der zweite Gelenkarm (1G, 2G) ausgebildet sind, wenigstens eine Versorgungsleitung zumindest teilweise jeweils entlang ihrer Längsachsen zu führen.

2. Computertomographie-Anlage (1) nach Anspruch 1, wobei der zweite Gelenkarm (2G) weiter über einen deckenständigen dritten Gelenkpunkt (3GP) oberhalb der Gantry (G) rotierbar angebunden ist.

3. Computertomographie-Anlage (1) nach Anspruch 1 oder 2, wobei die wenigstens eine Versorgungsleitung in der Vertikalsäule (VS) und an dem ersten, zweiten und/oder dritten Gelenkpunkt (1GP, 2GP, 3GP) in einer Energiekette (EK1, EK2, EK3) geführt ist.

4. Computertomographie-Anlage (1) nach einem der vorherigen Ansprüche, wobei der erste und der zweite Gelenkarm (1G, 2G) in einer Ebene verlaufen.

5. Computertomographie-Anlage (1) nach einem der vorherigen Ansprüche, wobei die Vertikalsäule (VS) höhenverstellbar ausgebildet ist und der erste, zweite und/oder dritte Gelenkpunkt (1GP, 2GP, 3GP) eine Verschwenkbewegung um eine Horizontalachse erlauben.

6. Computertomographie-Anlage (1) nach einem der vorherigen Ansprüche, wobei der zweite Gelenkpunkt (2GP) derart ausgebildet ist, dass er Stellungen erlaubt, in denen der erste Gelenkarm (1G) und der zweite Gelenkarm (2G) einen Winkel zwischen 10° und 170° einschließen.

7. Computertomographie-Anlage (1) nach einem der vorherigen Ansprüche, wobei der erste Gelenkpunkt (1GP) derart ausgebildet ist, dass er Stellungen erlaubt, in denen der erste Gelenkarm (1GP) und die Gantry (G) einen Winkel zwischen 10° und 160° einschließen.

8. Computertomographie-Anlage (1) nach einem der vorherigen Ansprüche, wobei die Länge des ersten Gelenkarms (1G) 65% bis 75% der Länge des zweiten Gelenkarms (2G) entspricht.

9. Computertomographie-Anlage (1) nach einem der vorherigen Ansprüche, wobei der erste Gelenkarm (1G) maximal 1600 mm und der zweite Gelenkarm (2G) maximal 2300 mm lang ist.

10. Computertomographie-Anlage (1) nach einem der vorherigen Ansprüche, ferner umfassend eine oberhalb der Gantry (G) verlaufende, deckenständige Horizontalsäule (HS), die sich in ihrer Längsachse parallel zu der Bewegungsrichtung (BR) der Gantry (G) erstreckt, wobei an der Horizontalsäule (HS) ein in Längsrichtung der Horizontalsäule (HS) verstellbarer Laufwagen (LW) angeordnet ist, der den dritten Gelenkpunkt (3GP) trägt.

11. Computertomographie-Anlage (1) nach Anspruch 10, wobei der Laufwagen (LW) ausgebildet ist, die Höhe des dritten Gelenkpunktes (3GP) zu verstellen.

12. Computertomographie-Anlage (1) nach Anspruch 10 oder 11, wobei die wenigstens eine Versorgungsleitung wenigstens teilweise in einer Energiekette (EK4, EK5) in der Horizontalsäule (HS) weitergeführt wird.

13. Computertomographie-Anlage (1) nach einem der Ansprüche 1 bis 12, wobei die Computertomographie-Anlage zur Erzeugung tomographischer Röntgenbilder ausgebildet ist.

14. Computertomographie-Anlage (1) zur Erzeugung tomographischer Röntgenbilder nach Anspruch 13, umfassend ein Schienensystem (SS), auf welchem die Gantry (G) über eine Strecke von maximal 12 m zwischen zwei Untersuchungsräumen (UR1, UR2) verstellbar ist.

15. Computertomographie-Anlage (1) zur Erzeugung tomographischer Röntgenbilder nach Anspruch 14, wobei die Gantry (G) ausgebildet ist, bei einer Verstellbewegung entlang des Schienensystems (SS) eine Rotation um eine durch das Isozentrum der Gantry (G) verlaufende Vertikalachse (VA) um 180° zu vollziehen.

## Claims

1. Computer tomography system (1) with a cable routing system (KS), wherein a gantry (G) of the computer tomography system (1) is designed so as to be able to be adjusted in a movement direction (BR) that extends perpendicular to the gantry (G),
the cable routing system (KS) comprising
- a vertical column (VS) that is arranged on the gantry (G) and extends vertically upward from a base of the computer tomography system (1) and
- a first articulated arm (1G) and also a second articulated arm (2G), wherein the first articulated arm (1G) is rotatably attached to an upper end of the vertical column (VS) above the gantry (G) via a first point of articulation (1GP) and is also rotatably attached to the second articulated arm (2G), which is likewise arranged above the gantry (G), via a second point of articulation (2GP), wherein the vertical column (VS) and the first and the second articulated arm (1G, 2G) are designed so as to route at least one supply line at least in part along their respective longitudinal axes.

2. Computer tomography system (1) according to claim 1, wherein the second articulated arm (2G) is further rotatably attached via a ceiling-mounted third point of articulation (3GP) above the gantry (G).

3. Computer tomography system (1) according to claim 1 or 2, wherein the at least one supply line is routed in the vertical column (VS) and on the first, second and/or third point of articulation (1GP, 2GP, 3GP) in a cable carrier (EK1, EK2, EK3).

4. Computer tomography system (1) according to one of the preceding claims, wherein the first and the second articulated arm (1G, 2G) extend in one plane.

5. Computer tomography system (1) according to one of the preceding claims, wherein the vertical column (VS) is designed in a height-adjustable manner and the first, second and/or third point of articulation (1GP, 2GP, 3GP) render possible a pivoting movement about a horizontal axis.

6. Computer tomography system (1) according to one of the preceding claims, wherein the second point of articulation (2GP) is designed in such a manner that it renders possible positions in which the first articulated arm (1G) and the second articulated arm (2G) include an angle between 10° and 170°.

7. Computer tomography system (1) according to one of the preceding claims, wherein the first point of articulation (1GP) is designed in such a manner that it renders possible positions in which the first articulated arm (1GP) and the gantry (G) include an angle between 10° and 160°.

8. Computer tomography system (1) according to one of the preceding claims, wherein the length of the first articulated arm (1G) corresponds to 65% to 75% of the length of the second articulated arm (2G).

9. Computer tomography system (1) according to one of the preceding claims, wherein the first articulated arm (1G) is maximum 1600 mm long and the second articulated arm (2G) is maximum 2300 mm long.

10. Computer tomography system (1) according to one of the preceding claims, further comprising a horizontal column (HS) that extends above the gantry (G), is ceiling-mounted and extends in its longitudinal axis parallel to the movement direction (BR) of the gantry (G), wherein a carriage (LW) that can be adjusted in the longitudinal direction of the horizontal column (HS) is arranged on the horizontal column (HS) and the carriage supports the third point of articulation (3GP).

11. Computer tomography system (1) according to claim 10, wherein the carriage (LW) is designed so as to adjust the height of the third point of articulation (3GP).

12. Computer tomography system (1) according to claim 10 or 11, wherein the at least one supply line is further routed at least in part in a cable carrier (EK4, EK5) in the horizontal column (HS).

13. Computer tomography system (1) according to one of claims 1 to 12, wherein the computer tomography system is designed so as to generate tomographic X-ray images.

14. Computer tomography system (1) for generating tomographic X-ray images according to claim 13, comprising a rail system (SS) on which the gantry (G) can be adjusted over a path of maximum 12 m between two examination spaces (UR1, UR2).

15. Computer tomography system (1) for generating tomographic X-ray images according to claim 14, wherein the gantry (g) is designed in the case of an adjustment movement along the rail system (SS) so as to perform a rotation by 180° about a vertical axis (VA), which extends through the isocentre of the gantry (G).

## Revendications

1. Installation (1) de tomodensitométrie assistée par ordinateur comprenant un système (KS) de guidage de câbles, dans laquelle un portique (G) de l'installation (1) de tomodensitométrie assistée par ordinateur est constitué de manière à pouvoir être déplacé dans une direction (BR) de mouvement s'étendant perpendiculairement au portique (G), le système (KS) de guidage de câbles comprenant :
- une colonne (VS) verticale, qui est montée sur le portique (G) et qui s'étend verticalement vers le haut à partir d'un socle de l'installation (1) de tomodensitométrie assistée par ordinateur, et
- un premier bras (1G) bras d'articulation ainsi qu'un deuxième bras (2G) bras d'articulation, dans laquelle le premier bras (1G) bras d'articulation peut, par un premier point (1GP) d'articulation, tourner à une extrémité supérieure de la colonne (VS) verticale au-dessus du portique (G), ainsi qu'être assemblé à rotation, par l'intermédiaire d'un deuxième point (2GP) d'articulation au deuxième bras (2G) bras d'articulation, monté également au-dessus du portique (G),
dans laquelle la colonne (VS) verticale et le premier et le deuxième bras (1G, 2G) bras d'articulation sont constitués pour guider au moins une ligne d'alimentation au moins en partie respectivement le long de leur axe longitudinal.

2. Installation (1) de tomodensitométrie assistée par ordinateur suivant la revendication 1, dans laquelle le deuxième bras (2G) bras d'articulation est assemblé à rotation au-dessus du portique (G) en outre par un troisième point (3GP) d'articulation au plafond.

3. Installation (1) de tomodensitométrie assistée par ordinateur suivant la revendication 1 ou 2, dans lequel la au moins une ligne d'alimentation passe en une chaîne (EK1, EK2, EK3) énergétique dans la colonne (VS) verticale et sur le premier, deuxième et/ou troisième points (1GP, 2GP, 3GP) d'articulation.

4. Installation (1) de tomodensitométrie assistée par ordinateur suivant l'une des revendications précédentes, dans laquelle le premier et le deuxième bras (1G, 2G) bras d'articulations s'étendent dans un plan.

5. Installation (1) de tomodensitométrie assistée par ordinateur suivant l'une des revendications précédentes, dans laquelle la colonne (VS) verticale est de constitution réglable en hauteur et le premier, deuxième et/ou troisième points (1GP, 2GP, 3GP) d'articulation autorise un mouvement de pivotement autour d'un axe horizontal.

6. Installation (1) de tomodensitométrie assistée par ordinateur suivant l'une des revendications précédentes, dans laquelle le deuxième point (2GP) d'articulation est constitué de manière à permettre des positions, dans lesquelles le premier bras (1G) d'articulation et le deuxième bras (2G) d'articulation font un angle entre 10° et 170°.

7. Installation (1) de tomodensitométrie assistée par ordinateur suivant l'une des revendications précédentes, dans laquelle le premier point (1GP) d'articulation est constitué de manière à permettre des positions, dans lesquelles le premier bras (1GP) d'articulation et le portique (G) font un angle compris entre 10° et 160°.

8. Installation (1) de tomodensitométrie assistée par ordinateur suivant l'une des revendications précédentes, dans laquelle la longueur du premier bras (1G) d'articulation représente de 65 à 75 % de la longueur du deuxième bras (2G) d'articulation.

9. Installation (1) de tomodensitométrie assistée par ordinateur suivant l'une des revendications précédentes, dans laquelle le premier bras (1G) d'articulation a une longueur de 1600 mm au maximum et le deuxième bras (2G) d'articulation de 2300 mm au maximum.

10. Installation (1) de tomodensitométrie assistée par ordinateur suivant l'une des revendications précédentes, comprenant, en outre, une colonne (HS) horizontale au plafond s'étendant au-dessus du portique (G), qui s'étend dans sa direction longitudinale parallèlement à la direction (BR) de déplacement du portique (G), dans laquelle, sur la colonne (HS) horizontale, est monté un chariot (LW), qui peut être déplacé dans la direction longitudinale de la colonne (HS) horizontale et qui porte le troisième point (3GP) d'articulation.

11. Installation (1) de tomodensitométrie assistée par ordinateur suivant la revendication 10, dans laquelle le chariot (LW) est constitué de manière à régler le niveau du troisième point (3GP) d'articulation.

12. Installation (1) de tomodensitométrie assistée par ordinateur suivant la revendication 10 ou 11, dans laquelle la au moins une ligne d'alimentation continue à passer au moins en partie en une chaîne (EK4, EK5) énergétique dans la colonne (HS) horizontale.

13. Installation (1) de tomodensitométrie assistée par ordinateur suivant l'une des revendications 1 à 12, dans laquelle l'installation de tomodensitométrie assistée par ordinateur est constituée pour la production d'images aux rayons X tomodensitométriques.

14. Installation (1) de tomodensitométrie assistée par ordinateur pour la production d'images de rayons X tomodensitométriques suivant la revendication 13, comprenant un système (SS) de rail, sur lequel le portique (G) peut être déplacé sur une distance de 12 m au maximum entre deux espaces (UR1, UR2) d'examen.

15. Installation (1) de tomodensitométrie assistée par ordinateur suivant la revendication 14, dans laquelle le portique (G) est constitué pour accomplir, lors d'un mouvement de déplacement le long du système (SS) de rail, une rotation de 180° autour d'un axe (VA) vertical passant par l'isocentre du portique (G).
